# EUROPÄISCHE PATENTSCHRIFT

(11) **EP 0 471 983 B1**
(45) Veröffentlichungstag und Bekanntmachung des Hinweises auf die Patenterteilung: **22.03.1995**
(21) Anmeldenummer: 91111914.7
(22) Anmeldetag: 17.07.1991
(51) Int. Cl.: C07C 275/06, C07C 273/18

(54) **Verfahren zur N-Alkylierung von Harnstoffen**
Process for the N-alkylation of urea
Procédé de N-alkylation d'urée

(30) Priorität: 14.08.1990 AT 1691/90
(43) Veröffentlichungstag der Anmeldung: 26.02.1992
(73) Patentinhaber: Chemie Linz GmbH, A-4021 Linz (AT)
(72) Erfinder: Hackl, Kurt Alfred, Dipl.-Ing., A-4020 Linz (AT); Falk, Heinz, Dr., A-4040 Linz (AT)
(74) Vertreter: Kunz, Ekkehard, Dr.

(56) Entgegenhaltungen:
- EP-A- 0 427 963
- CHEMICAL ABSTRACTS, Band 110, Nr. 15, 10. April 1989, Seite 675, Zusammenfassung Nr. 134854e, Columbus, Ohio, US; N. AYYANGAR: "A non-phosgene route for the synthesis of sym-N,N'-diethyldiphenylurea"
- CHEMICAL ABSTRACTS, Band 100, Nr. 21, 21. Mai 1984, Seite 631, Zusammenfassung Nr. 174798q, Columbus, Ohio, US; A BOGATSKY et al: "Macroheterocycles. XIX. A convenient synthesis of N-alkylated thioureas"

## Beschreibung

Die Erfindung betrifft ein Verfahren zur Herstellung von N-alkylierten Harnstoffen durch Umsetzung eines Harnstoffes mit einem Alkylierungsmittel.

N-alkylierte Harnstoffe mußten bisher über Umwege, nämlich z.B. über die Herstellung eines Amins, das dem gewünschten Harnstoff entsprechend substituiert werden mußte, und das dann mit dem Harnstoff unter Austausch eines Harnstoffaminteils, oder mit einem entsprechenden Isocyanat oder Carbamoylchlorid umgesetzt wurde, hergestellt werden.

So ist in R.A. Jacobson, J.Am.Chem.Soc. 58, 1984 (1936) beschrieben, daß der Versuch, Harnstoffe durch Umsetzung von Mononatriumharnstoffen mit Alkylhalogeniden am Stickstoffatom zu alkylieren, nicht erfolgreich verlief.
Aus P.A. Ongley, Trans.Proc.Roy.Soc., New Zealand 77, 10 (1948) geht hervor, daß bei der Alkylierung von Harnstoff mit Alkylsulfaten Alkylisoharnstoffe und nicht N-alkylierte Harnstoffe entstehen.
In F. Korte: Methodicum Chimicum, Band 6, 716 und 732, Georg Thieme Verlag, Stuttgart, 1974 werden diese Ergebnisse bestätigt. Dort ist beschrieben, daß Harnstoffe bei der Umsetzung mit Alkylhalogeniden stets am Sauerstoffatom und nicht am Stickstoffatom alkyliert werden, und daß bei der Alkylierung von Harnstoffen mit Dialkylsulfaten oder p-Toluolsulfonsäureestern Isoharnstoffe entstehen.
U. Petersen und E. Kühle in E. Müller: Methoden der Organischen Chemie (Houben-Weyl); 4. Auflage, Band E4, 335ff und 594, Georg Thieme Verlag Stuttgart - New York, 1983 und die dort zitierte Literatur bestätigen ebenfalls die ausschließliche O-Alkylierbarkeit von Harnstoffen.

Aus Chemistry and Industry, Vol. 18, Sept. 1988, Seiten 599 bis 600 ist die symmetrische N-Alkylierung von N,N'-Diphenylharnstoff bekannt, wobei die Alkylierung bei Verwendung eines Phasentransferkatalysators stets an beiden N-Atomen des Ausgangsharnstoffes stattfindet, sodaß ein symmetrisch alkylierter Harnstoff erhalten wird.
Eine direkte unsymmetrisch an nur einem N-Atom stattfindende N-Alkylierung wurde bisher jedoch noch nicht beschrieben.

Es wurde nun unerwarteterweise gefunden, daß Harnstoffe unsymmetrisch an nur einem Stickstoffatom alkyliert werden können, wenn man den Harnstoff in Gegenwart eines Phasentransferkatalysators und einer Base mit einem Alkylierungsmittel umsetzt.

Gegenstand der Erfindung ist daher ein Verfahren zur Herstellung von Harnstoffen der Formel 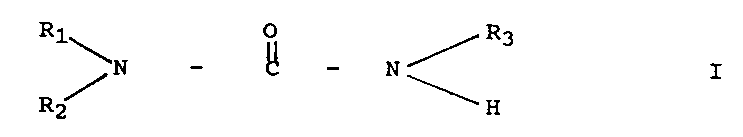
in der R₁ und R₂ unabhängig voneinander Wasserstoff, eine geradkettige, verzweigte oder cyclische, unsubstituierte oder durch unter den Reaktionsbedingungen inerte Gruppen substituierte Alkylgruppe, eine Aralkylgruppe oder R₁ und R₂ gemeinsam mit dem Stickstoffatom einen nicht aromatischen, heterocyclischen Ring und R₃ eine unsubstituierte oder durch unter den Reaktionsbedinungen inerte Gruppen substituerte Alkylgruppe, wobei die tertiäre Butylgruppe ausgeschlossen ist, bedeutet, dadurch gekennzeichnet, daß ein Harnstoff der Formel 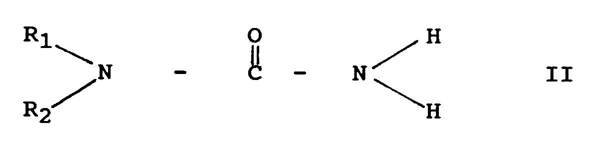
in der R₁ und R₂ die obgenannte Bedeutung haben in Gegenwart einer festen Base und in Gegenwart eines Phasentransferkatalysators in einem Verdünnungsmittel bei Temperaturen von 0 bis 150 °C mit einem Alkylierungsmittel der Formel

(R₃)ₙ - X III

in der R₃ die oben angegebene Bedeutung hat und n die Zahlen 1 oder 2 bedeutet, wobei für den Fall, daß n die Zahl 1 bedeutet, X für ein Halogenid, eine Sulfonsäure- oder Hydrogensulfatgruppe und für den Fall, daß n die Zahl 2 bedeutet, X für eine Sulfatgruppe steht, N-alkyliert wird.

Als Harnstoff wird ein Harnstoff der Formel 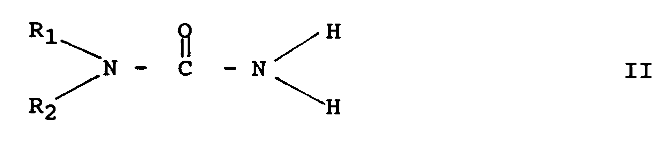
eingesetzt, worin R₁ und R₂ unabhängig voneinander Wasserstoff, eine geradkettige, verzweigte oder cyclische unsubstituierte oder durch unter den Reaktionsbedingungen inerte Gruppen substiutierte Alkylgruppe oder eine Aralkylgruppe bedeuten, oder R₁ und R₂ bedeuten gemeinsam mit dem Stickstoffatom einen nichtaromatischen heterocyclischen Ring.

Unter Alkylgruppen sind dabei Alkylgruppen mit 1 bis 22, bevorzugt mit 2 bis 18 C-Atomen zu verstehen, wie z.B. Ethyl-, Propyl-, iso-Propyl-, tert.Butyl-, iso-Pentyl-, Methylcyclopentyl-, Cyclohexyl-, 3-Ethylhexyl-, Octyl-, Decyl-, Dodecyl-, Hexadecyl-, Octadecylgruppen.
Die Alkygruppen können unsubstituiert oder durch unter den Reaktionsbedingungen inerte Gruppen, wie etwa durch Fluoratome, Nitrogruppen, Alkenyl-, Alkyliden-, Arylgruppen, Alkoxygruppen mit 1 bis 5 C-Atomen, z.B. Methoxy-, Ethoxy-, iso-Propoxy-, Butoxygruppen oder Phenoxygruppen substituiert sein. Bevorzugt sind die Alkylgruppen unsubstitiert.

Unter Aralkylgruppen sind Benzyl- oder Phenylethylgruppen, wobei die Phenylgruppen durch unter den Reaktionsbedingungen inerte Gruppen, wie Alkylgruppen mit 1 bis 5 C-Atomen, z.B. Ethyl-, iso-Propyl-, iso-Pentylgruppen, Alkoxygruppen mit 1 bis 5 C-Atomen, z.B. Methoxy-, Ethoxy-, iso-Propxy-, Butoxygruppen, Halogenide wie Fluor, Chlor, Brom oder Nitrogruppen substituiert sein können, zu verstehen.
R₁ und R₂ können auch gemeinsam mit dem Stickstoffatom einen nicht aromatischen, heterocyclischen Ring bilden, also z.B. einen Pyrrolidin-, Piperidin-, Morpholinring, 1,4-Thiazan.

Bevorzugt bedeuten R₁ und R₂ unabhängig voneinander Wasserstoff, eine unsubstituierte, geradkettige oder verzweigte Alkylgruppe mit 2 bis 10 C-Atomen oder R₁ und R₂ bedeuten gemeinsam mit dem Stickstoffatom einen nicht aromatischen, heterocyclischen Ring, bevorzugt den Pyrrolidin- oder Morpholinring.

Verbindungen der Formel II können entweder mit Hilfe des erfindungsgemäßen Verfahrens hergestellt werden, oder sie sind nach einem der üblichen, bekannten Verfahren, etwa durch Umsetzung von Harnstoff oder Isocyansaure mit einem entsprechenden Amin herstellbar.

Als Basen kommen feste Basen, wie Alkalihydroxide, z.B. Kaliumhydroxid, Natriumhydroxid oder Alkaliamide z.B. Natriumamid oder Kaliumamid in Frage. Bevorzugt werden Alkalihydroxide eingesetzt, wobei es vorteilhaft ist, wenn das Alkalihydroxid einen geringen Gehalt eines Carbonates wie Kaliumcarbonat, Natriumcarbonat, der 2 bis 20 Mol.% bezogen auf das Alkalihydroxid beträgt, aufweist. Die Base wird in fester, gepulverter Form oder in Form von Pellets im Überschuß verwendet. Bevorzugt werden pro Mol Harnstoff der Formel II 1,5 bis 8 Mol, besonders bevorzugt 3 bis 5 Mol der festen Base eingesetzt.

Als Katalysator kommen übliche Phasentransferkatalysatoren in Frage oder Dimethylsulfoxid. Eine Zusammenfassung von verwendbaren Phasentransferkatalysatoren und ihr möglicher Einsatz in verschiedenen Verdünnungsmitteln ist in W.E. Keller: Phasetransfer reactions (Fluka Compendium), Vol. 1 u. 2; Georg Thieme Verlag Stuttgart - New York, 1986 u. 1987) geoffenbart. Bevorzugt werden als Phasentransferkatalysatoren quaternäre Ammoniumsalze, wie z.B. Tetrabutylammoniumhydrogensulfat, Tetrabutylammoniumchlorid oder Benzyltriethylammoniumchlorid eingesetzt. Wird Harnstoff selbst als Harnstoff der Formel II eingesetzt, wird Dimethylsulfoxid als Katalysator verwendet. Die jeweilige Auswahl des Katalysators erfolgt nach dem jeweils verwendeten Verdünnungsmittel bzw. nach dem jeweils verwendeten Ausgangsprodukt.

Als Alkylierungsmittel wird eine Verbindung der Formel

(R₃)ₙ - X III

eingesetzt, in der R₃ eine unsubstituierte oder durch unter den Reaktionsbedingungen inerte Gruppen substituierte Alkylgruppe, wobei die tert.Butylgruppe ausgeschlossen ist, und n die Zahl 1 oder 2 bedeutet, und wobei für den Fall, daß n die Zahl 1 bedeutet, X für ein Halogenid, eine Sulfonsäure- oder Hydrogensulfatgruppe, und für den Fall, daß n die Zahl 2 bedeutet, X für eine Sulfatgruppe steht. Unter Halogenid ist dabei Chlorid, Bromid oder Jodid zu verstehen. Bevorzugt bedeutet X in der Formel III ein Halogenid, eine Sulfonsäuregruppe oder eine Sulfatgruppe.
Unter Alkylgruppe sind die oben angeführten Alkylgruppen zu verstehen, die unsubstituiert oder durch unter den Reaktionsbedingungen inerte Gruppen substituiert sein können, wie sie oben angeführt sind, wobei die tert.Butylgruppe ausgeschlossen ist. Das Alkylierungsmittel wird im allgemeinen äquimolar zum Harnstoff der Formel II eingesetzt, wobei aber im Einzelfall ein Überschuß des einen oder anderen Reaktionsteilnehmers möglich sein kann.

Als Verdünnungsmittel werden unter den Rekationsbedingungen inerte Verdünnungsmittel, welche Lösungsmittel für den Harnstoff der Formel II und das Alkylierungsmittel sind, eingesetzt. Es sind dies aromatische Kohlenwasserstoffe, z.B. Benzol, Toluol, Xylol, höhere aliphatische Kohlenwasserstoffe, z.B. Paraffine, aromatische halogenierte Kohlenwasserstoffe z.B. Chlorbenzol, Trichlorbenzole, Ether, z.B. Tetrahydrofuran oder Dimethylsulfoxid oder Mischungen solcher Verdünnungsmittel. Bevorzugt werden aromatische Kohlenwasserstoffe oder Dimethylsulfoxid, besonders bevorzugt Toluol oder Dimetylsulfoxid eingesetzt. Wenn Dimethylsulfoxid als Verdünnungsmittel verwendet wird, dient es gleichzeitig auch als Katalysator. Die eingesetzte Base soll in dem verwendeten Verdünnungsmittel nicht vollständig löslich sein. Ist das Ausgangsprodukt der Formel II Harnstoff, wird Dimethylsulfoxid als Verdünnungsmittel verwendet.

Zur Durchführung des erfindungsgemäßen Verfahrens wird zuerst der Harnstoff der Formel II in dem Verdünnungsmittel gelöst und vorgelegt. Bevorzugt wird das Verdünnungsmittel vor dem Einsatz vorgetrocknet. Sodann wird die feste Base in Form von Pellets oder in gepulverter Form zugesetzt und durch starkes Rühren gut suspendiert, worauf der Katalysator und das Alkylierungsmittel zugegeben werden. Es hat sich herausgestellt, daß es beim Einsatz von Dimethylsulfoxid als Katalysator und Verdünnungsmittel günstig sein kann, zuerst die pelletierte oder gepulverte, feste Base im Dimethylsulfoxid gut zu suspendieren und danach den Harnstoff der Formel II und das Alkylierungsmittel einzurühren, wobei als Alkylierungsmittel in diesem Fall bevorzugt ein Alkylhalogenid eingesetzt wird.

Die Reaktionsmischung wird stark gerührt und gegebenenfalls auf Temperaturen bis zu 150 °C erhitzt. Bevorzugt wird auf 70 bis 150 °C, besonders bevorzugt auf Rückflußtemperatur des jeweils verwendeten Verdünnungsmittels erhitzt. Im Fall der Verwendung von Dimethylsulfoxid als Katalysator und Verdünnungsmittel wird nicht auf Rückfluß erhitzt; in diesem Fall wird eine Temperatur von O bis 100 °C, bevorzugt von 20 bis 70 °C angewendet.

Dabei bildet sich der Harnstoff der Formel I.

Nach beendeter Umsetzung wird Wasser zugesetzt und der Harnstoff der Formel I mit Hilfe eines Extraktionsionsmittels aus der Reaktionsmischung extrahiert. Als Extraktionsmittel werden mit Wasser nicht mischbare, organische Extraktionsmittel, wie halogenierte Kohlenwasserstoffe, beispielsweise Methylenchlorid, Chloroform, oder Ether beispielsweise Diethylether, Diisopropylether eingesetzt. Die organische Phase wird mit Wasser gewaschen, getrocknet und das Verdünnungsmittel verdampft, wobei eine Nachtrocknung im Vakuum erfolgen kann. Im allgemeinen ist die Reinheit des auf diese Weise hergestellten Harnstoffes der Formel I ausreichend. Gegebenenfalls kann auch noch ein Reinigungsschritt z.B. durch Umkristallisieren, Chromatographie oder Destillation angeschlossen werden.

In einer bevorzugten Ausführungsform wird Ethyl-, iso-Propyl-, Butyl-, tert.Butyl-, Decylharnstoff oder Pyrrolidin- oder Morpholincarbonsäureamid in Toluol gelöst, mit 3 bis 5 Äquivalenten Kalium- oder Natriumhydroxidpellets, die 4 bis 10 Mol% Kalium- oder Natriumkarbonat enthalten, sowie mit 0,04 bis 0,06 Äquivalenten eines quaternären Ammoniumsalzes als Phasentransferkatalysator unter starkem Rühren versetzt und auf Rückfluß erhitzt. Nach beendeter Reaktion wird die Reaktionsmischung mit Wasser versetzt und mit Methylenchlorid und/oder Chloroform mehrmals extrahiert. Die vereinigten organischen Phasen werden mit Wasser gewaschen, getrocknet und das Verdünnungsmittel verdampft, worauf im Vakuum nachgetrocknet wird.

In einer anderen bevorzugten Ausführungsform werden 3 bis 5 Äquivalente gepulvertes Kaliumhydroxid in Dimethylsulfoxid suspendiert und mit je einem Äquivalent Harnstoff und Alkylhalogenid versetzt. Die Reaktionsmischung wird bei Temperaturen von 20 bis 70 °C gerührt, nach beendeter Reaktion mit Wasser versetzt und mit Methylenchlorid und/oder Chloroform extrahiert. Die vereinigten organischen Phasen werden getrocknet und das Verdünnungsmittel verdampft, worauf eine Nachtrocknung im Ölpumpenvakuum erfolgt.

Auf die beschriebene Art und Weise werden alkylierte Harnstoffe ohne Umweg in guten Ausbeuten und guter Reinheit hergestellt. Das Verfahren stellt somit eine Bereicherung der Technik dar.

### Beispiel 1

2,32 g N-Butylharnstoff (20 mMol), 3,2 g NaOH-Pellets (80 mMol), 0,55 g Kaliumcarbonat (4 mMol) und 280 mg (1 mMol) Tetrabutylammoniumchlorid wurden in 40 ml Toluol suspendiert und mit 2,18 g Ethylbromid (20 mMol) unter starkem Rühren versetzt. Die Reaktionsmischung wurde 2 Stunden auf Rückfluß erhitzt, mit 150 ml destilliertem Wasser versetzt und mit 50 ml Chloroform und 50 ml Methylenchlorid extrahiert. Die vereinigten organischen Phasen wurden mit Wasser gewaschen, über Natriumsulfat getrocknet und abgedampft. Der Rückstand wurde im Vakuum nachgetrocknet.
Dabei wurden 2,16 g, das sind 76 % der Theorie, **N-Butyl-N'-ethylharnstoff** erhalten.

¹H-NMR (CDCl₃; 200 MHz; delta): 5,874 (s breit; 2H; NH); 3,220-3,125 (m(dt und dq); 4H; N-CH₂); 1,460-1,320 (m(tt und tq); 4H; Butyl-CH₂-CH₂); 1,112 (t; 3H; Ethyl-CH₃; J_{E} = 6,5 Hz); 0,858 (t; 3H; Butyl-CH₃; J_{B} = 6,5 Hz) ppm.

### Beispiel 2

Wie im Beispiel 1 beschrieben, aber unter Verwendung von 2,46 g Propylbromid als Alkylierungsmittel wurde **N-Butyl-N'-propylharnstoff** in einer Ausbeute von 78 % der Theorie hergestellt.

¹H-NMR (CDCl₃; 200 MHz; delta): 5,864 und 5,836 (2t; je 1H; NH); 3,104 (2dt; je 2H; N-CH₂; J_{CH2NH} = 6,0 Hz; J_{CH2-CH2} = 7,4 Hz); 1,542-1,349 (m(tt und 2tq); 6H; Butyl-CH₂-CH₂ und Propyl-CH₂); 0,906 (t; 6H; Butyl-CH₃ und Propyl-CH₃; J = 7,3 Hz) ppm.

### Beispiel 3

Wie im Beispiel 1 beschrieben, aber unter Verwendung von 4,5 g KOH-Pellets (80 mMol) und 0,55 g Kaliumcarbonat (4 mMol) als Basen und unter 16 Stunden Erhitzen auf Rückfluß wurde **N-Butyl-N'-ethylharnstoff** in einer Ausbeute von 52 % der Theorie erhalten.
Das ¹H-NMR-Spektrum stimmte mit dem von Beispiel 1 völlig überein.

### Beispiel 4

Wie im Beispiel 1 beschrieben, aber unter Verwendung von 1,54 g Diethylsulfat (10 mMol) als Alkylierungsmittel wurde **N-Butyl-N'-ethylharnstoff** in einer Ausbeute von 80 % der Theorie erhalten.
Das ¹H-NMR-Spektrum stimmte mit dem von Beispiel 1 völlig überein.

### Beispiel 5

Wie im Beispiel 1 beschrieben, aber unter Verwendung von 4,0 g Toluol-4-sulfonsäureethylester (20 mMol) als Alkylierungsmitttel wurde **N-Butyl-N'-ethylharnstoff** in einer Ausbeute von 74 % der Theorie erhalten.
Das ¹H-NMR-Spektrum stimmte mit dem von Beispiel 1 völlig überein.

### Beispiel 6

Wie im Beispiel 1 beschrieben, aber unter Verwendung von 2,48 g Methansulfonsäurethylester (20 mMol) als Alkylierungsmittel wurde **N-Butyl-N'-ethylharnstoff** in einer Ausbeute von 71 % der Theorie erhalten.
Das ¹H-NMR-Spektrum stimmte mit dem von Beispiel 1 völlig überein.

### Beispiel 7

Wie im Beispiel 1 beschrieben, aber unter Verwendung von 1,76 g N-Ethylharnstoff (20 mMol) als Harnstoff und 2,74 g Butylbromid (20 mMol) als Alkylierungsmittel wurde **N-Butyl-N'-ethylharnstoff** in einer Ausbeute von 71 % der Theorie erhalten.
Das ¹H-NMR-Spektrum stimmte mit dem von Beispiel 1 völlig überein.

### Beispiel 8

Wie im Beispiel 1 beschrieben, aber unter Verwendung von 2,04 g N-Isopropylharnstoff (20 mMol) als Harnstoff und 2,74 g Butylbromid (20 mMol) als Alkylierungsmittel wurde **N-Butyl-N'-isopropylharnstoff** in einer Ausbeute von 58 % hergestellt.

¹H-NMR (CDCl₃; 200 MHz; delta): 5,708 (t; 1H; Butyl-NH; J_{CH2NH} = 5,5 Hz); 5,490 (d; 1H; Isopropyl-NH; J_{CHNH} = 7,9 Hz), 3,858 (dq; 1H; Isopropyl-CH; J_{CHNH} = 7,9 Hz; J_{CHCH3} = 6,5 Hz); 3,135 (dse; 2H; Butyl-N-CH₂; J_{CH2NH} = 5,5 Hz; J_{CH2CH2} = 6,7 Hz); 1,498-1,275 (m(tt und tq); 4H; Butyl-CH₂-CH₂); 1,122 (d; 6H; Isopropyl-CH₃; J_{CHCH3} = 6,5 Hz); 0,906 (t; 3H; Butyl-CH₃; J_{CH2CH3} = 7,0 Hz) ppm.

### Beispiel 9

Wie im Beispiel 1 beschrieben, aber unter Verwendung von 2,46 g 2-Brompropan (20 mMol) als Alkylierungsmittel wurde **N-Butyl-N'-isopropylharnstoff** in einer Ausbeute von 35 % der Theorie hergestellt.
Das ¹H-NMR-Spektrum stimmte mit dem von Beispiel 8 völlig überein.

### Beispiel 10

Wie im Beispiel 1 beschrieben, aber unter Verwendung von 2,74 g Butylbromid (20 mMol) als Alkylierungsmittel wurde **N,N'-Dibutylharnstoff** in einer Ausbeute von 82 % der Theorie erhalten.

¹H-NMR (CDCl₃; 200 MHz; delta): 5,914 (t; 2H; NH; J_{CH2NH} = 5,9 Hz); 3,135 (dt; 4H; N-CH₂; J_{CH2NH} = 5,9 Hz; J_{CH2CH2} = 6,7 Hz); 1,497-1,315 (m(tt und tq); 8H; Butyl-CH₂-CH₂); 0,908 (t; 6H; CH₃); J_{CH2CH3} = 7,0 Hz) ppm.

### Beispiel 11

Wie im Beispiel 10 beschrieben, aber unter Verwendung von 4,5 g KOH-Pellets (80 mMol) und 0,55 g Kaliumcarbonat (4 mMol) als Base und 12 Stunden Erhitzen auf Rückfluß wurde **N,N'-Dibutylharnstoff** in einer Ausbeute von 75 % der Theorie erhalten.
Das ¹H-NMR-Spektrum stimmte mit dem von Beispiel 10 völlig überein.

### Beispiel 12

Wie im Beispiel 1 beschrieben, aber unter Verwendung von 1,85 g Butylchlorid (20 mMol) als Alkylierungsmittel und 4,5 g KOH-Pellets (80 mMol) und 0,55 g Kaliumcarbonat (4 mMol) als Base und 12 Stunden Erhitzen auf Rückfluß wurde **N,N'-Dibutylharnstoff** in einer Ausbeute von 67 % der Theorie erhalten.
Das ¹H-NMR-Spektrum stimmte mit dem von Beispiel 10 völlig überein.

### Beispiel 13

Wie im Beispiel 1 beschrieben, aber unter Verwendung von 1,85 g Butylchlorid (20 mMol) als Alkylierungsmittel wurde **N,N'-Dibutylharnstoff** in einer Ausbeute von 21 % der Theorie erhalten.
Das ¹H-NMR-Spektrum stimmte mit dem von Beispiel 10 völlig überein.

### Beispiel 14

Wie im Beispiel 1 beschrieben, aber unter Verwendung von 2,32 g tert.Butylharnstoff (20 mMol) als Harnstoff und 2,74 g Butylbromid (20 mMol) als Alkylierungsmittel wurde **N-Butyl-N'-tert.Butylharnstoff** in einer Ausbeute von 47 % der Theorie erhalten.

¹H-NMR (CDCl₃; 200 MHz; delta): 5,421 (t; 1H; Butyl-NH; J_{CH2NH} = 6,0 Hz); 5,239 (s; 1H; tert.Butyl-NH); 3,093 (dt; 2H; N-CH₂; J_{CH2NH} = 6,0 Hz; J_{CH2CH2} = 6,3 Hz); 1,436-1,371 (m(tt und tq); 4H; Butyl-CH₂-CH₂); 1,311 (s; 9H; tert.Butyl-CH₃); 0,896 (t; 3H; Butyl-CH₃; J_{CH2CH3} = 6,9 Hz) ppm.

### Beispiel 15

Wie im Beispiel 1 beschrieben, aber unter Verwendung von 4,47 g Decylbromid (20 mMol) als Alkylierungsmittel wurde **N-Butyl-N'-decylharnstoff** nach dem Umkristallisieren aus Diethylether in einer Ausbeute von 96 % der Theorie erhalten.

¹H-NMR (CDCl₃; 200 MHz; delta): 5,531 (t; 2H; NH; J_{CH2NH} = 5,1 Hz); 3,161-3,072 (m(2dt); 4H; N-CH₂; J_{CH2NH} = 5,1 Hz); 1,522-1,256 (M; 20H; Butyl- und Decyl-CH₂); 0,941 (t; 3H; Butyl-CH₃; J_{CH2CH3(B)} = 6,0 Hz); 0,896 (t; 3H; Decyl-CH₃; J_{CH2CH3(D)} = 6,6 Hz) ppm.

### Beispiel 16

Wie im Beispiel 15 beschrieben, aber unter Verwendung von 3,54 g Decylchlorid (20 mMol) als Alkylierungsmittel wurde **N-Butyl-N'-Decylharnstoff** in einer Ausbeute von 95 % der Theorie erhalten.
Das ¹H-NMR-Spektrum stimmte mit dem von Beispiel 15 völlig überein.

### Beispiel 17

Wie im Beispiel 1 beschrieben, aber unter Verwendung von 4,0 g N-Decylharnstoff (20 mMol) und 4,47 g Decylbromid wurde nach dem Umkristallisieren aus n-Hexan **N,N,-Didecylharnstoff** in einer Ausbeute von 97 % der Theorie erhalten.

¹H-NMR (CDCl₃; 200 MHz; delta): 4,524 (t; 2H, NH, J_{CH2NH} = 6,0 Hz); 3,139 (dt; 4H, N-CH₂; J_{CH2NH} = 6,0 Hz; J_{CH2CH2} = 6,8 Hz);

1,481 (tt; 4H; N-CH₂-CH₂; J_{CH2CH2} = 6,8 Hz); 1,259 (m; 32H; Decyl-CH₂); 0,880 (t; 6H; CH₃, J_{CH2CH3} = 6,5 Hz) ppm.

### Beispiel 18

Wie im Beispiel 17 beschrieben, aber unter Verwendung von 3,54 g Decylchlorid (20 mMol) als Alkylierungsmittel wurde N,N'-Didecylharnstoff in einer Ausbeute von 95 % der Theorie erhalten.
Das ¹H-NMR-Spektrum stimmte mit dem von Beispiel 17 völlig überein.

### Beispiel 19

Wie im Beispiel 1 beschrieben, aber unter Verwendung von 2,28 g Pyrrolidincarbonsäureamid (20 mMol) als Harnstoff und 2,74 g Butylbromid (20 mMol) als Alkylierungsmittel wurde **N-Pyrrolidincarbonsäurebutylamid** in einer Ausbeute von 41 % der Theorie erhalten.

¹H-NMR (CDCl₃; 200 MHz; delta): 4,390 (t; 1H; NH; J_{CH2NH} = 5,8 Hz); 3,340 (t; 4H; N-CH₂; J₁₂ = 6,7 Hz); 3.222 (dt; 2H; HN-CH₂; J_{CH2NH} = 5,8 Hz; J_{CH2CH2} = 7,0 Hz); 1,893 (tt; 4H; Pyrrolidin-CH₂-CH₂; J₁₂ = 6,7 Hz; J₂₃ = 3,5 Hz); 1,536-1,288 (m(dt und tq); 4H; Butyl-CH₂-CH₂); 0,921 (t; 3H; CH₃; J_{CH2CH3} = 7,1 Hz) ppm.

### Beispiel 20

Wie im Beispiel 19 beschrieben, aber unter Verwendung von 4,5 g KOH-Pellets (80 mMol) und 0,55 g Kaliumcarbonat (4 mMol) als Base und unter 16 Stunden Erhitzen auf Rückfluß wurde **N-Pyrrolidincarbonsäurebutylamid** in einer Ausbeute von 70 % der Theorie erhalten.
Das ¹H-NMR-Spektrum stimmte mit dem von Beispiel 19 völlig überein.

### Beispiel 21

Wie im Beispiel 1 beschrieben, aber unter Verwendung von 2,60 g Morpholincarbonsäureamid (20 mMol) als Harnstoff und 2,46 g Propylbromid (20 mMol) als Alkylierungsmittel wurde **N-Morpholincarbonsäurepropylamid** in einer Ausbeute von 60 % der Theorie erhalten.

¹H-NMR (CDCl₃; 200 MHz; delta): 4,992 (t; 1H; NH; J_{CH2NH} = 5,8 Hz); 3.634 (t; 4H; O-CH₂; J = 5,0 Hz); 3,308 (t; 4H; N-CH₂; J = 5,0 Hz); 3,132 (dt; 2H; HN-CH₂; J_{CH2NH} = 5,8 Hz; J_{CH2CH2} = 7.3 Hz); 1,476 (tq; 2H; HN-CH₂-CH₂; J_{CH2CH2} = 7,3 Hz; J_{CH2CH3} = 7,3 Hz); 0,870 (t; 3H; CH₃; J_{CH2CH3} = 7,3 Hz) ppm.

### Beispiel 22

Wie im Beispiel 21 beschrieben, aber unter Verwendung von 2,46 g 2-Brompropan wurde **N-Morpholincarbonsäureisopropylamid** in einer Ausbeute von 26 % der Theorie erhalten.

¹H-NMR (CDCl₃; 200 MHz; delta): 4,555 (d; 1H; NH; J_{CHNH} = 6,7 Hz); 3.969 (dse; 1H; HN-CH; J_{CHNH} = 6,7 Hz; J_{CHCH3} = 6,6 Hz); 3,675 (t; 4H, O-CH₂; J = 4,9 Hz); 3,328 (t; 4H; N-CH₂; J = 4,9 Hz); 1.153 (d; 6H; CH₃; J_{CHCH3} = 6,6 Hz) ppm.

### Beispiel 23

Wie im Beispiel 21 beschrieben, aber unter Verwendung von 2,74 g Butylbromid (20 mMol) als Alkylierungsmittel wurde **N-Morpholincarbonsäurebutylamid** in einer Ausbeute von 85 % der Theorie erhalten.

¹H-NMR (CDCl₃; 200 MHz; delta): 5,413 (t; 1H; NH; J_{CH2NH} = 6,0 Hz); 3,664 (t; 4H; O-CH₂; J = 5,0 Hz); 3,360 (t; 4H; N-CH₂; J = 5,0 Hz); 3,190 (dt; 2H; HN-CH₂; J_{CH2NH} = 6,0 Hz; J_{CH2CH2} = 6,7 Hz); 1.522-1.275 (m(tt und tq); 4H; Butyl-CH₂-Ch₂); 0,917 (t; 3H; CH₃; J_{CH2CH3} = 6,7 Hz) ppm.

### Beispiel 24

Wie im Beispiel 21 beschrieben, aber unter Verwendung von 4,47 g Decylbromid (20 mMol) wurde nach dem Umkristallisieren aus n-Hexan **Morpholincarbonsäuredecylamid** in einer Ausbeute von 88 % der Theorie erhalten.

¹H-NMR (CDCl₃; 200 MHz; delta): 4,787 (t; 1H; NH; J_{CH2NH} = 5,6 Hz); 3.678 (t; 4H; O-CH₂; J = 5,0 Hz); 3,340 (t; 4H; N-CH₂; J = 5,0 Hz); 3,208 (dt; 2H; HN-CH₂; J_{CH2NH} = 5,6 Hz; J_{CH2CH2} = 7,2 Hz); 1,493 (tt; 2H; HN-CH₂-CH₂; J_{CH2CH2} = 7,2 Hz); 1,261 (m; 14 H; Decyl-CH₂); 0,880 (t; 3H, CH₃, J_{CH2CH3} = 6,5 Hz) ppm.

### Beispiel 25

2,25 g gepulverte KOH (40 mMol) wurden unter Argon in 20 ml trockenem Dimethylsulfoxid suspendiert. Nach 10 Minuten wurde unter starkem Rühren 0,6 g Harnstoff (10 mMol) und 2,12 g Hexyljodid (10 mMol) zugegeben und noch weitere 30 Minuten beim Raumtemperatur gerührt. Nach beendeter Reaktion wurde die Reaktionsmischung in 150 ml destilliertes Wasser gegossen und die entstandene Suspension mit Methylenchlorid und Chloroform extrahiert. Die organische Phase wurde mit Wasser gewaschen, über Natriumsulfat getrocknet und abgedampft.
Dabei wurden 0,33 g **N-Hexylharnstoff**, das sind 26 % der Theorie, erhalten.

¹H-NMR (CDCl₃; 200 MHz; delta): 5,895 (t; 1H; NH; J_{CH2NH} = 5,3 Hz); 5.359 (s; 2H, NH₂); 2.914 (dt; 2H; N-CH₂; J_{CH2NH} = 5,3 Hz; J_{CH2CH2} = 6,4 Hz); 1.312-1.230 (m; 8H; Hexyl-CH₂); 0,846 (t; 3H; CH₃; J = 6,5 Hz) ppm.

### Beispiel 26

Wie im Beispiel 25 beschrieben, aber unter Verwendung von 1,16 g Butylharnstoff (10 mMol) als Harnstoff und 1,37 g Butylbromid (10 mMol) als Alkylierungsmittel wurde **N,N'-Dibutylharnstoff** in einer Ausbeute von 22 % der Theorie erhalten.

Das ¹H-NMR-Spektrum stimmte mit dem von Beispiel 10 völlig überein.

## Patentansprüche

1. Verfahren zur Herstellung von Harnstoffen der Formel 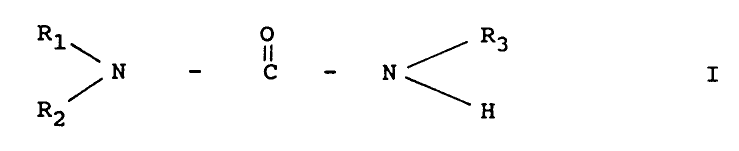 in der R₁ und R₂ unabhängig voneinander Wasserstoff, eine geradkettige, verzweigte oder cyclische, unsubstituierte oder durch unter den Reaktionsbedingungen inerte Gruppen substituierte Alkylgruppe, eine Aralkylgruppe oder R₁ und R₂ gemeinsam mit dem Stickstoffatom einen nicht aromatischen, heterocyclischen Ring und R₃ eine unsubstituierte oder durch unter den Reaktionsbedinungen inerte Gruppen substituerte Alkylgruppe, wobei die tertiäre Butylgruppe ausgeschlossen ist, bedeutet, dadurch gekennzeichnet, daß ein Harnstoff der Formel 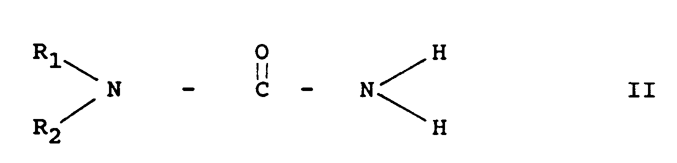 in der R₁ und R₂ die obgenannte Bedeutung haben, in Gegenwart einer festen Base und in Gegenwart eines Phasentransferkatalysators in einem Verdünnungsmittel bei Temperaturen von 0 bis 150 °C mit einem Alkylierungsmittel der Formel
(R₃)ₙ - X III
in der R₃ die oben angegebene Bedeutung hat, und n die Zahlen 1 oder 2 bedeutet, wobei für den Fall, daß n die Zahl 1 bedeutet, X für ein Halogenid, eine Sulfonsäure- oder Hydrogensulfatgruppe und für den Fall, daß n die Zahl 2 bedeutet, X für eine Sulfatgruppe steht, N-alkyliert wird.

2. Verfahren nach Anspruch 1, dadurch gekennzeichnet, daß ein Harnstoff der Formel II, in der R₁ und R₂ unabhängig voneinander Wasserstoff, eine unsubstituierte, geradkettige oder verzweigte Alkylgruppe mit 2 bis 18 C-Atomen oder R₁ und R₂ gemeinsam mit dem Stickstoffatom einen Pyrrolidin-, Piperidin- oder Morpholinring bedeuten, eingesetzt wird.

3. Verfahren nach einem der Ansprüche 1 oder 2, dadurch gekennzeichnet, daß als Base ein festes Alkalihydroxid mit oder ohne Zusatz von 2 bis 20 Mol% festem Alkalicarbonat, bezogen auf das Alkalihydroxid eingesetzt wird.

4. Verfahren nach einem der Ansprüche 1 bis 3, dadurch gekennzeichnet, daß pro Mol Harnstoff der Formel II 1,5 bis 8 Mol der festen Base eingesetzt werden.

5. Verfahren nach einem der Ansprüche 1 bis 4, dadurch gekennzeichnet, daß als Phasentransferkatalysator ein quaternäres Ammoniumsalz eingesetzt wird.

6. Verfahren nach einem der Ansprüche 1 bis 5, dadurch gekennzeichnet, daß als Verdünnungsmittel ein aromatischer Kohlenwasserstoff eingesetzt wird.

7. Verfahren nach einem der Ansprüche 1 bis 4, dadurch gekennzeichnet, daß als Verdünnungsmittel und als Katalysator Dimethylsulfoxid eingesetzt wird.

8. Verfahren nach einem der Ansprüche 1 bis 6, dadurch gekennzeichnet, daß die Umsetzung bei Rückflußtemperatur des Verdünnungsmittels ausgeführt wird, sofern nicht Dimethylsulfoxid als Verdünnungsmittel eingesetzt wird.

9. Verfahren nach einem der Ansprüche 1 bis 8, dadurch gekennzeichnet, daß ein Alkylierungsmittel der Formel III eingesetzt wird, in dem R₃ eine geradkettige oder verzweigte unsubstituierte Alkylgruppe und X ein Chlorid, Bromid, eine 4-Toluolsulfonat-, Methylsulfonat- oder eine Sulfatgruppe bedeutet.

10. Verfahren nach einem der Ansprüche 1 bis 9, dadurch gekennzeichnet, daß der Harnstoff der Formel II und das Alkylierungsmittel der Formel III äquimolar eingesetzt werden.

## Claims

1. Process for the preparation of ureas of the formula 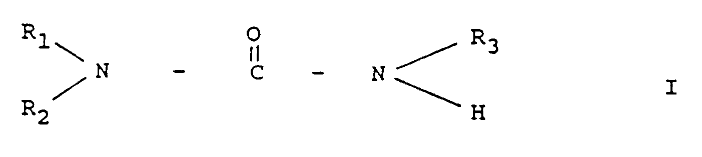 in which R₁ and R₂ independently of one another denote hydrogen, a linear, branched or cyclic alkyl group which is unsubstituted or substituted by groups inert under the conditions of the reaction, or an aralkyl group, or R₁ and R₂, together with the nitrogen atom, denote a non-aromatic, heterocyclic ring and R₃ denotes an alkyl group which is unsubstituted or substituted by groups inert under the conditions of the reaction, the tertiary butyl group being excluded, characterized in that a urea of the formula 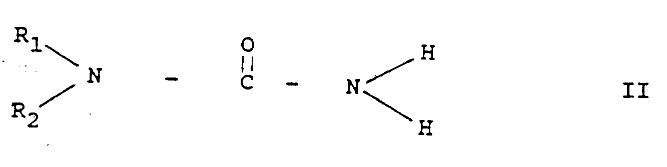 in which R₁ and R₂ have the abovementioned meaning is N-alkylated in the presence of a solid base and in the presence of a phase transfer catalyst in a diluent at temperatures of 0 to 150°C with an alkylating agent of the formula
(R₃)ₙ - X III
in which R₃ has the meaning indicated above and n denotes the numbers 1 or 2, X representing a halide or a sulphonic acid or bisulphate group in the event that n denotes the number 1, and X representing a sulphate group in the event that n denotes the number 2.

2. Process according to Claim 1, characterized in that a urea of the formula II in which R₁ and R₂ independently of one another denote hydrogen or an unsubstituted, linear or branched alkyl group having 2 to 18 C atoms, or R₁ and R₂, together with the nitrogen atom, denote a pyrrolidine, piperidine or morpholine ring, is employed.

3. Process according to one of Claims 1 or 2, characterized in that the base employed is a solid alkali metal hydroxide with or without the addition of 2 to 20 mol% of a solid alkali metal carbonate, relative to the alkali metal hydroxide.

4. Process according to one of Claims 1 to 3, characterized in that 1.5 to 8 mol of the solid base are employed per mole of urea of the formula II.

5. Process according to one of Claims 1 to 4, characterized in that a quaternary ammonium salt is employed as the phase transfer catalyst.

6. Process according to one of Claims 1 to 5, characterized in that an aromatic hydrocarbon is employed as the diluent.

7. Process according to one of Claims 1 to 4, characterized in that dimethyl sulphoxide is employed as the diluent and as the catalyst.

8. Process according to one of Claims 1 to 6, characterized in that the reaction is carried out at the reflux temperature of the diluent, unless dimethyl sulphoxide is employed as the diluent.

9. Process according to one of Claims 1 to 8, characterized in that an alkylating agent of the formula III in which R₃ denotes a linear or branched, unsubstituted alkyl group and X denotes a chloride or bromide or a 4-toluenesulphonate, methylsulphonate or sulphate group, is employed.

10. Process according to one of Claims 1 to 9, characterized in that the urea of the formula II and the alkylating agent of the formula III are employed in an equimolar ratio.

## Revendications

1. Procédé de préparation d'urées de formule : 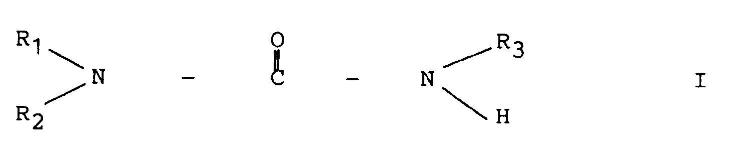 où R₁ et R₂ signifient, indépendamment l'un de l'autre, un atome d'hydrogène, un groupe alkyle linéaire, ramifié ou cyclique, non substitué ou substitué par des groupes inertes dans les conditions de réaction, ou un groupe aralkyle, ou R₁ et R₂ pris avec l'atome d'azote signifient un noyau hétérocyclique non aromatique, et R₃ signifie un groupe alkyle non substitué ou substitué par des groupes inertes dans les conditions de réaction, à l'exclusion du groupe tert-butyle, caractérisé en ce qu'on effectue la N-alkylation d'une urée de formule : 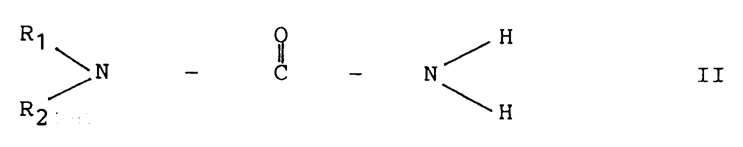 où R₁ et R₂ ont la signification indiquée ci-dessus, en présence d'une base solide et en présence d'un catalyseur de transfert de phase, dans un diluant, à des températures de 0 à 150°C, avec un agent d'alkylation de formule :
(R₃)n - X III
où R₃ a la signification indiquée ci-dessus, et n signifie 1 ou 2, et, pour le cas où n signifie le chiffre 1, X représente un halogénure, un groupe sulfo ou un groupe hydrogénosulfate, et pour le cas où n signifie le chiffre 2, X représente un groupe sulfate.

2. Procédé selon la revendication 1, caractérisé en ce qu'on utilise une urée de formule II où R₁ et R₂ signifient, indépendamment l'un de l'autre, un atome d'hydrogène ou un groupe alkyle non substitué à chaîne droite ou ramifiée, ayant de 2 à 18 atomes de carbone, ou R₁ et R₂ pris avec l'atome d'azote signifient un noyau de pyrrolidine, de pipéridine ou de morpholine.

3. Procédé selon la revendication 1 ou 2, caractérisé en ce qu'on utilise, comme base, un hydroxyde alcalin solide, avec ou sans l'addition de 2 à 20 % en moles de carbonate alcalin solide, par rapport à l'hydroxyde alcalin.

4. Procédé selon l'une des revendications 1 à 3, caractérisé en ce qu'on utilise de 1,5 à 8 modes de base solide par mole d'urée de formule II.

5. Procédé selon l'une des revendications 1 à 4, caractérisé en ce qu'on utilise un sel d'ammonium quaternaire comme catalyseur de transfert de phase.

6. Procédé selon l'une des revendications 1 à 5, caractérisé en ce qu'on utilise un hydrocarbure aromatique comme diluant.

7. Procédé selon l'une des revendications 1 à 4, caractérisé en ce qu'on utilise le diméthylsulfoxyde comme diluant et comme catalyseur.

8. Procédé selon l'une des revendications 1 à 6, caractérisé en ce qu'on effectue la réaction à la température de reflux du diluant quand on n'utilise pas le diméthylsulfoxyde comme diluant.

9. Procédé selon l'une des revendications 1 à 8, caractérisé en ce qu'on utilise un agent d'alkylation de formule III où R₃ signifie un groupe alkyle non substitué, à chaîne droite ou ramifiée, et X signifie un chlorure, un bromure, un groupe 4-toluènesulfonate, un groupe méthylsulfonate ou un groupe sulfate.

10. Procédé selon l'une des revendications 1 à 9, caractérisé en ce qu'on utilise des quantités équimolaires d'urée de formule II et d'agent d'alkylation de formule III.
